# EUROPEAN PATENT APPLICATION

(11) **EP 4 311 557 A1**
(43) Date of publication of application: **31.01.2024**
(21) Application number: 22382713.0
(22) Date of filing: 26.07.2022
(51) Int. Cl.: A61K 39/395, A61P 35/00, C07K 5/02, C07K 14/315, C07K 16/40

(54) **FAP-TARGETED ANTIBODY-DRUG CONJUGATES**

(71) Applicant: Oncomatryx Biopharma, S.L., 48160 Derio (Vizcaya) (ES)
(72) Inventor: Fabre, Myriam, Derio (ES); Ferrer, Cristina, Derio (ES); García Ribas, Ignacio, Derio (ES); Moreno Ruiz, Pablo, Derio (ES); Simon, Laureano, Derio (ES)
(74) Representative: Mewburn Ellis LLP

(57) **Abstract**

The present invention relates to anti-Fibroblast Activating Protein α (FAP)-targeted antibody-drug conjugates (ADCs), in particular anti-FAP antibody-cytolysin conjugates having the formula A-(L-D)p, or a pharmaceutically acceptable salt or solvate thereof, wherein A is an anti-FAP antibody that selectively binds FAP, L is a linker, D is a drug comprising a cytolysin and p is 1 to 10, for use in a method of treatment of FAP+ sarcoma in a mammalian subject. *In vitro* methods of selecting sarcoma patients for treatment with a FAP-targeted ADC are also disclosed.

## Description

### Field of the Invention

The present invention relates to antibody drug conjugates (ADCs) and immunotoxins that target Fibroblast Activating Protein α (FAP), and to their use in the treatment of sarcoma. The invention also relates to the use of FAP as a biomarker for the selection of sarcoma patients to be treated with a FAP-targeted ADC.

### Background

A sarcoma is a type of cancer that starts in tissues like bone or muscle. Bone and soft tissue sarcoma consist of a heterogenous group of rare solid tumours of mesenchymal origin. Sarcoma tumours arise predominantly from the embryonic mesoderm that can affect patients from any age in any part of the body. Despite the progress in the therapy of these tumours, metastasis and death remain a significant issue in patients with high-risk soft tissue sarcoma (Cormier et al. (2004), Guedes et al (2021), Niederhuber et al (2014)).

These solid tumors frequently exhibit significant stromal reactions such as the so-called "desmoplastic stroma" or "reactive stroma", which represents 20-60% of total tumor mass and is characterized by the existence of large numbers of stromal cells and dense extracellular matrix (ECM). Recent studies have indicated the tumor-promoting roles of stromal cells, as exemplified by vascular cells, immune cells, fibroblasts, myofibroblasts, adipocytes and bone marrow-derived progenitors (Weinberg, et al. (2007), Nieman, et al. (2011), Joyce, et al. (2009), Hanahan, et al. (2012), Gupta, et al (2006), Valastyan, et al (2011)). In particular, considerable numbers of cancer-associated fibroblasts (CAFs) are frequently observed within tumor-associated stroma of various human cancers, including breast, lung, colon, and pancreas carcinomas (Kalluri, et al. (2006), Pietras, et al. (2010)). Interacting coordinately with the different components of the stroma, CAFs have the ability to promote neoangiogenesis and tumor growth. CAFs have also been shown as crucial for the development of aggressive tumors and tumor invasiveness during cancer progression (Orimo, et al. (2005), Erez, et al. (2010), Olumi, et al. (1999), Yang, et al. (2006), Hwang, et al. (2008), Hu, et al. (2009), Medema, et al. (2011), Malanchi, et al. (2012), Strell, et al. (2012), Horimoto, et al. (2012)). CAFs facilitate the spreading and infiltration of tumor cells in distant organs, thus contributing to formation of metastases. Importantly, the relevance of stromal cells to the failure of systemic drug delivery to tumors and to the development of drug resistance has also been indicated (Meads, et al. (2009), Olive, et al. (2009), Acharyya, et al. (2012), Crawford, et al. (2009), Straussman, et al (2012)).

Leiomyosarcoma (LMS) is a malignant mesenchymal tumour, derived from smooth muscle. It is one of the most frequent subtypes of sarcoma, representing up to 25% of all soft tissue sarcomas (Kannan (2022)). LMS has a propensity for metastasis via hematogenous dissemination. As smooth muscle is found in many areas of the body, leiomyosarcoma can develop in a wide range of tissues, such as the uterus (30% of all LMS), stomach, small intestine and retroperitoneum (35% of all LMS). Some studies have indicated that uterine LMS and non-uterine LMS display distinct disease biology.

Rhabdomyosarcoma is another malignant mesenchymal tumour. It is the most common soft tissue sarcoma in children, representing up to 50% of all pediatric soft tissue sarcomas and 3% of all pediatric tumours (Amer (2019)). It is divided into six histological groups: embryonal, alveolar, spindle cell, mixed-type, pleomorphic, and rhabdomyosarcoma with ganglionic differentiation.

Undifferentiated Pleomorphic Sarcoma (UPS) is a term that refers to and undifferentiated and unclassified sarcoma. This subtype consists of poorly differentiated tumor cells that may present as spindle-shaped cells, histiocytes and giant cells.

Monoclonal antibody (MAb)-based drugs represent a great promise in the fight against cancer. This is because they allow the treatment to be aimed at a molecular level in a precise and specific way. These advantages, together with their commercial appeal (short development times, restricted competence and being easily exportable to other cancer types once they have been approved), have pushed many pharmaceutical companies to invest heavily in the development of new antibody-based molecules, as well as in the in-licensing of new molecules or technologies from biotech companies.

However, despite the clinical success of therapeutic antibodies, naked MAbs targeting cell surface tumor antigens rarely present sufficient efficacy on their own. To increase the low activity of the MAbs, novel strategies are focusing on binding them to toxic molecules. Plant and bacterial toxins as well as small chemotherapeutic molecules can be good candidates, since they are very potent and active in very small quantities.

The field of immunotoxins (ITs) and Antibody-Drug conjugates (ADCs) for the treatment of cancer has recently experienced a growing development activity by pharmaceutical companies, due to the technological advances performed during the last years, aimed at solving the problems they initially presented about immunogenicity, undesirable toxicity, production, half-life and resistance.

Immunoconjugates are made of a human, humanized or chimeric recombinant antibody, covalently linked to a cytotoxic drug. The main goal of such a structure is joining the power of small cytotoxic agents (300 to 1000 Da) and the high specificity of tumor-associated antigen (TAA)- targeted MAbs.

The Ab must be very selective to reach the antigen, whose expression must be restricted to normal cells. The Ab also must be internalized efficiently into the cancerous cells.

The cytotoxic agent selected as the effector moiety must kill cells only after internalization and release into the cell cytoplasm. The most commonly used payloads in ADCs are DNA-harming drugs such as calicheamicins, duocarmicins, or microtubule-targeting compounds like auristatins and maytansinoids.

The Ab-cytotoxic linkers are designed to be stable systemically and to release the cytotoxic agent within the target cells.

TAAs are frequently cell membrane proteins that are overexpressed in diseased tissues or at least expressed sufficiently to facilitate the internalization-activated cytotoxicity. Ideally the antigen presents a restricted expression in normal tissues with a low or absent expression in vital organs. On top of this, the tumor antigen must be recognized selectively and with high affinity by an Ab.

In many types of human cancer, fibroblast response is characterized by the induction of a cell surface protein, Fibroblast Activating Protein α (FAPα), a serine protease of 95 kDa whose expression is highly restricted to developing organs, wound-healing and tissue remodeling. FAPα is a well-studied membrane glycoprotein type II serine protease, up-regulated in cancer associated fibroblasts (CAFs), as well as in wound-healing fibroblasts (Ramirez-Montagut et al. (2004)). In several tumour tissues, FAP expression has been associated with tumour proliferation, invasiveness, angiogenesis, epithelial-tomesenchymal transition (EMT), immunosuppression, and drug resistance (Xin et al. (2021)). FAP presents the following characteristics:
- Type II membrane glycoprotein with SER-protease activity (collagenase + DPP)
- 89% human-murine protein homology
- Tumor stroma-expressed in >90% carcinomas (breast, pancreas, lung, bladder and colon)
- Transitory and highly restricted expression in normal adult tissues during wound-healing and developing organs.
- FAP is expressed in tumour cells of various sarcomas, including leiomyosarcoma and rhabdomyosarcoma.
- FAP(+) fibroblasts located close to tumor vasculature
- Very focal expression
- Internalization
- Implication in extracellular matrix remodeling, tumor growth and metastasis.

FAP expression has been found in Pancreas tumor cells as well as tumor-associated stromal fibroblasts. FAP expression was correlated with shorter patient survival and worse prognosis, suggesting a possible FAP-based autocrine/paracrine loop in this type of tumor (Shi, et al. (2012)).

Previous work by Kontermann and Pfizenmaier (IZI, University of Stuttgart, Germany) developed anti-FAP MAb derivatives against both human and murine proteins (Mersmann, et al (2001), Brocks, et al. (2001)). They have shown *in vitro* that anti-FAP scFv immunoliposomes bind specifically FAP+ cells and get internalized (Schmidt, et al. (2001)). They demonstrated the anti-tumoral effect of nanoparticles covered with lipids and anti-FAP scFvs and loaded with TNFα (Messerschmidt, et al. (2009)).

Treatment with murine MAb FAP5-DM1 immunotoxin induced long lasting inhibition of tumor growth and full regression in pancreas and lung cancer xenograft models, without any intolerance-related effect (Ostermann, et al. (2008)).

FAP protein has been extensively validated as an interesting target for anticancer therapy in solid tumour and has therefore been subjected to various CAF-targeting approaches (Kakarla et al (2012), Simková et al.(2020)). In this context, OMTX705, a novel, FAP (+) CAF-targeting antibody-drug conjugate was developed as an alternative stroma-targeted therapeutic compound with a potent antineoplastic efficacy. Preclinical studies in patient-derived xenografted murine models of pancreas cancer, NSCLC, triple negative breast cancer and diffuse gastric cancer, have shown that FAP expression in these models is associated with the anti-tumoral efficacy of OMTX705 treatment (Fabre et al. (2020)).

First isolated from reactive stroma in human sarcomas (Rettig et al. (1994), Rettig et al. (1988)), FAP was also found expressed in tumour cells of bone and tissue sarcomas subsets (Rettig et al. (1993)), in association with their mesenchymal origin. Most recent studies about FAP protein as cancer biomarker in sarcomas evidenced some correlation between FAP and poor prognosis in osteosarcomas (Zhang et al. (2019)). Furthermore, the development of several ⁶⁸Ga-radiolabelled FAP inhibitor small-molecules has resulted in the emergence of studies evaluating its clinical role in cancer imaging (Kuyumcu et al. (2021)). Studies of tumour uptake quantification on ⁶⁸Ga-FAPI PET/CT in different types of primary and metastatic tumours, revealed that sarcoma patients were included in the group of the highest ⁶⁸Ga-FAPI ligand uptake. These high ratios resulted in high image contrast and excellent tumour delineation in bone and soft tissue sarcoma patients (Kratochwil et al. (2019), Koerber et al. (2021), Kessler et al. (2022)). Moreover, FAP-targeted peptides have been effective in radionuclide therapy for various cancers, such as ¹⁷Lu-FAP-2286, and in imaging (such as ⁶⁸Ga-FAP-2286) (Kwan et al. (2021)).

Despite these advances, there remains an unmet need for further therapeutic strategies for the treatment of sarcomas, including leiomyosarcoma, rhabdomyosarcoma and UPS, and for components for use in such therapeutic strategies. The present invention addresses these and other needs.

### Summary of the Invention

Broadly, the present invention relates to anti-FAP antibodies, conjugates thereof and optimised payloads for use in antibody conjugate strategies. In particular, the present inventors have found that anti-FAP antibodies as described herein exhibit highly specific binding, and fast and efficient internalisation. The invention provides anti-FAP antibody-cytolysin conjugates, of the type disclosed in WO 2015/118030 (incorporated herein by reference in its entirety), for use in a method of treatment of sarcoma in a mammalian subject.

The cytolysin derivatives described herein are advantageously conjugated to anti-FAP antibodies for use in the treatment of sarcoma, wherein the sarcoma is characterised by FAP expression in the tumour. Thus, the use of FAP-targeted ADCs is expanded beyond tumours which express FAP in the stroma.

Accordingly, in a first aspect the present invention provides an anti-Fibroblast Activating Protein α (FAP) antibody-cytolysin conjugate having the formula I:

A-(L-D)ₚ (I)

or a pharmaceutically acceptable salt or solvate thereof, wherein A is an anti-FAP antibody that selectively binds FAP, L is a linker, D is a drug comprising a cytolysin, and p is 1 to 10, for use in a method of treatment of sarcoma in a mammalian subject, wherein the sarcoma is characterised by FAP expression in the tumour.

In some cases, in accordance with this and other aspects of the present invention, the anti-FAP antibody is a monoclonal antibody or binding fragment thereof that selectively binds to an extracellular region of human FAP and/or murine FAP. In some cases, the anti-FAP antibody may cross-react to both human and murine FAP.

In particular cases the anti-FAP antibody may comprise heavy chain complementarity determining regions 1-3 (CDRH1-3) and light chain complementarity determining regions 1-3 (CDRL1-3) having the following amino acid sequences:
(i) CDRH1: SEQ ID NO: 7;
(ii) CDRH2: SEQ ID NO: 8;
(iii) CDRH3: SEQ ID NO: 9;
(iv) CDRL1: SEQ ID NO: 10;
(v) CDRL2: SEQ ID NO: 11; and
(vi) CDRL3: SEQ ID NO: 12.

In some embodiments, the CDRH1 region may comprise a variant of SEQ ID NO: 7 having up to 1 or 2 amino acid substitutions compared with the sequence of SEQ ID NO: 7. In some embodiments, the CDRH2 region may comprise a variant of SEQ ID NO: 8 having up to 1 or 2 amino acid substitutions compared with the sequence of SEQ ID NO: 8. In some embodiments, the CDRH3 region may comprise a variant of SEQ ID NO: 9 having up to 1 or 2 amino acid substitutions compared with the sequence of SEQ ID NO: 9. In some embodiments, the CDRL1 region may comprise a variant of SEQ ID NO: 10 having up to 1 or 2 amino acid substitutions compared with the sequence of SEQ ID NO: 10. In some embodiments, the CDRL2 region may comprise a variant of SEQ ID NO: 11 having up to 1 or 2 amino acid substitutions compared with the sequence of SEQ ID NO: 11. In some embodiments, the CDRL3 region may comprise a variant of SEQ ID NO: 12 having up to 1 or 2 amino acid substitutions compared with the sequence of SEQ ID NO: 12.

In certain cases, CDRH1-3 comprise the amino acid sequences of SEQ ID NOS: 7-9, respectively and CDRL1-3 comprise the amino acid sequences of SEQ ID NOS: 10-12, respectively.

In certain cases, the anti-FAP antibody comprises a heavy chain variable region (VH) comprising the amino acid sequence of SEQ ID NO: 5 and a light chain variable region (VL) comprising the amino acid sequence of SEQ ID NO: 6. In some embodiments, the heavy chain variable region (VH) has at least 90%, 95% or 99% sequence identity with the full-length sequence of SEQ ID NO: 5. In some cases, the light chain variable region (VL) has at least 90%, 95% or 99% sequence identity with the full-length sequence of SEQ ID NO: 6.

In certain cases, the anti-FAP antibody comprises a heavy chain comprising the amino acid sequence of SEQ ID NO: 1 and a light chain comprising the amino acid sequence of SEQ ID NO: 2. In some cases, the heavy chain has at least 90%, 95% or 99% sequence identity with the full-length sequence of SEQ ID NO: 1. In some cases, the light chain has at least 90%, 95% or 99% sequence identity with the full-length sequence of SEQ ID NO: 2.

In certain cases, the anti-FAP antibody comprises a heavy chain comprising the amino acid sequence of SEQ ID NO: 3 and a light chain comprising the amino acid sequence of SEQ ID NO: 4. In some cases, the heavy chain has at least 90%, 95% or 99% sequence identity with the full-length sequence of SEQ ID NO: 3. In some cases, the light chain has at least 90%, 95% or 99% sequence identity with the full-length sequence of SEQ ID NO: 4.

In certain cases, A may be a competitively binding anti-FAP antibody that is structurally different from the anti-FAP antibody molecules exemplified herein. For example, A may be an anti-FAP antibody molecule that competes with the anti-FAP IgG1 antibody identified herein as "hu36" for binding to immobilized recombinant human FAP. hu36 has the heavy chain amino acid sequence of SEQ ID NO: 3 and the light chain amino acid sequence of SEQ ID NO: 4. The anti-FAP antibody may, in some case, bind to the same epitope as hu36. Methods for determining antibody binding competition and for epitope mapping are well known in the art, see for example "Epitope Mapping by Competition Assay" Ed Harlow and David Lane, Cold Spring Harb Protoc; 2006; doi:10.1101/pdb.prot4277.

In accordance with this and other aspects of the present invention, D may be a cytolysin. The cytolysin may, in some cases, be a compound disclosed in WO 2008/138561 A1, the entire contents of which is expressly incorporated herein by reference (compounds disclosed therein are also referred to as Tubulysine derivatives). The cytolysin may be synthesised as described in WO 2008/138561. In certain cases, the cytolysin may be as defined in Formula I or Formula IV of WO 2008/138561 A1. In certain cases, the cytolysin may be of formula IV: 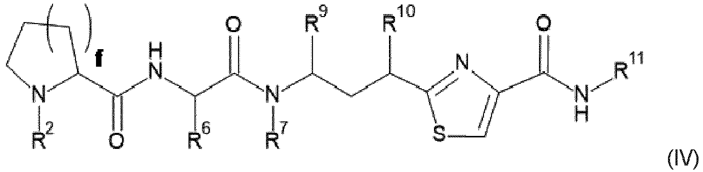 wherein:
R² is H or is C₁-C₄ alkyl;
R⁶ is C1-C6 alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is H, OH, O-alkyl or O-acetyl;
f is 1 or 2;
R¹¹ has the following structure: 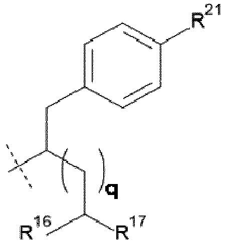 wherein
R²¹ is H, OH, halogen, NH₂, alkyloxy, phenyl, alkyl amino or dialkyl amino;
R¹⁶ is H or a C₁-C₆-alkyl group;
R¹⁷ is directly or indirectly attached to linker L; and
q is 0, 1, 2 or 3;
and wherein the term "optionally substituted" relates to groups, wherein one or several H atoms can be replaced by F, Cl, Br or I or OH, SH, NH₂, or NO₂; the term "optionally substituted" further relates to
groups, which can be exclusively or additionally substituted with unsubstituted C₁-C₆ alkyl, C₂C₆ alkenyl, C₂-C₆ alkynyl, C₁-C₆ heteroalkyl, C₃-C₁₀ cycloalkyl, C₂-C₉ heterocycloalkyl, C₆-C₁₀ aryl, C₁-C₉ heteroaryl, C₇-C₁₂ aralkyl or C₂-C₁₁ heteroaralkyl groups.

In some cases R² is a bond to linker L.

In some cases R¹⁷ is C(O)X, CONHNHX, OX, NHX or SX, wherein X is a bond to linker L.

In some embodiments, linker L comprises a spacer. In some cases the spacer has a chain length of 2 to 30 atoms. In some cases the spacer comprises or consists of an alkylene (i.e. divalent alkyl) or heteroalkylene (i.e. divalent heteroalkyl) group. In some cases the spacer comprises or consists of an alkylene or oxyalkylene group.

In some embodiments, the spacer comprises -(OCH₂CH₂)ₙ-, wherein n is 2 to 5. In some cases the spacer comprises or consists of a group -(CH₂)ₙ- or -(OCH₂CH₂)ₙ-, wherein n ≥ 1. In some cases the spacer comprises or consists of a group -(OCH₂CH₂)ₙ-, wherein n ≥ 1. In particular, n may be 1 to 15, 1 to 10, 1 to 6, or 2 to 5. For example, n may be 3 or 4. In some cases the spacer comprises between one and six ethylene glycol units, e.g. a triethylene glycol. In some cases the spacer may be directly attached to group R¹⁷, or may be attached to group R¹⁷ via a bridging group. In some cases the spacer is attached to group R¹⁷ via a -C(O)X bridging group, wherein X is a bond to R¹⁷. In some cases R¹⁷ is CONHNHX and the spacer is attached to group R¹⁷ via a -C(O)X bridging group, wherein X represents the bond between the spacer and R¹⁷. In some cases R¹⁷ is CONHNHX and the spacer is a -(OCH₂CH₂)ₙ- attached to R¹⁷ via a -C(O)X bridging group, wherein n = 2, 3 or 4.

In some embodiments, L comprises an attachment group for attachment to A.

In some embodiments, L comprises a protease cleavable portion comprising a valine-citrulline unit. For example, L may comprise maleimidocaproyl-valine-citrulline-p-aminobenzylcarbamate.

In some cases the cytolysin has the following structure: 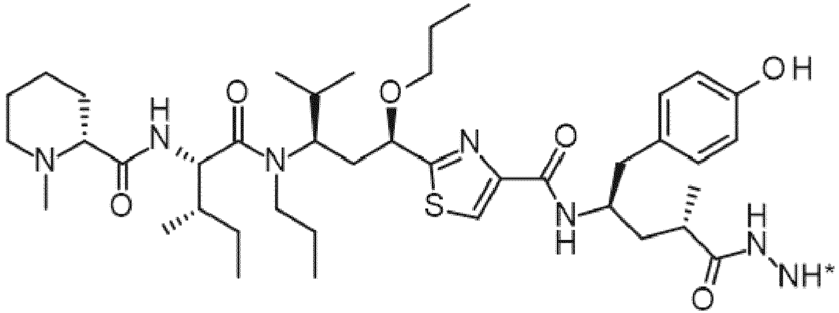 wherein * indicates the site of attachment to L.

In some cases D comprises a cytolysin having the following structure:

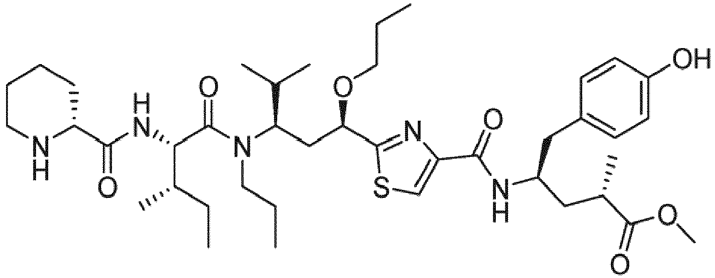

In some cases the double bond of the maleimide is reacted with a thiol group of a cysteine residue of the antibody A to form a sulphur-carbon bond in order to effect linkage of the linker L to the antibody A.

In some embodiments -L-D has the structure:

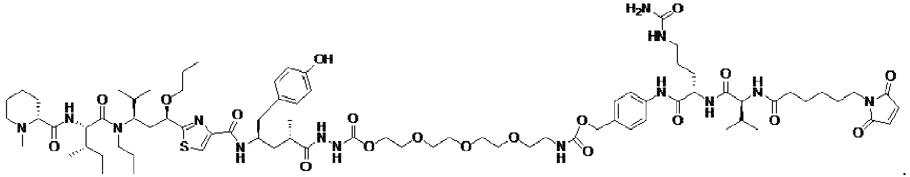

In other cases -L-D has a structure selected from the group consisting of: 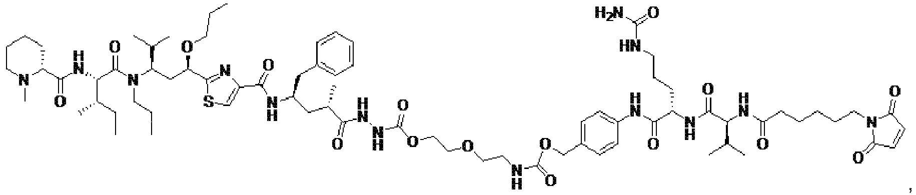 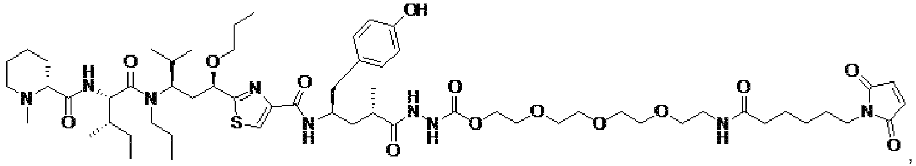 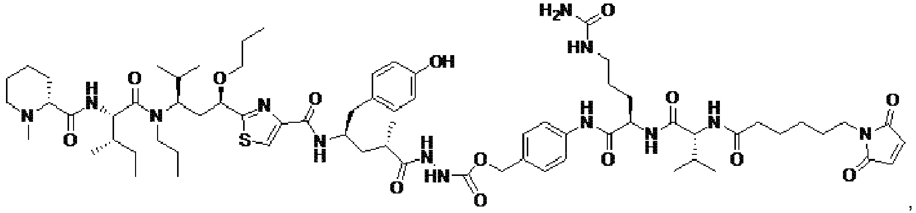 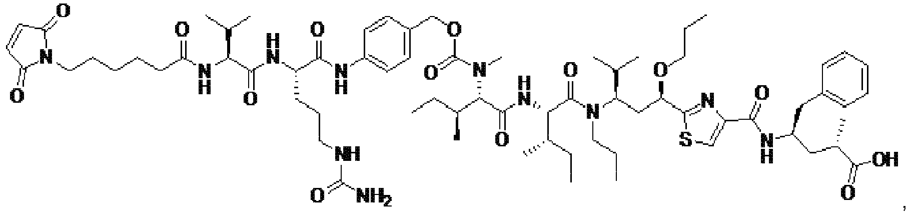 and

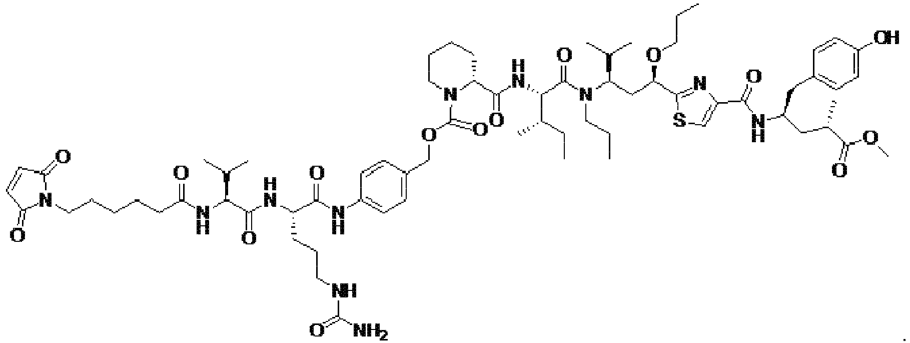

In certain cases -L-D may have the following structure:

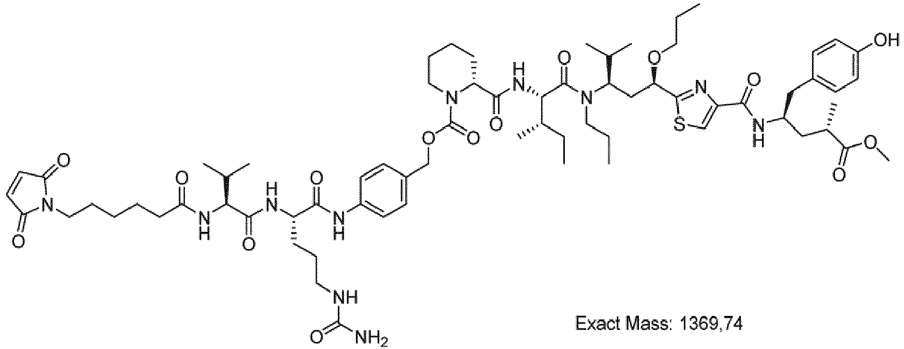

In certain cases -L-D may have the following structure:

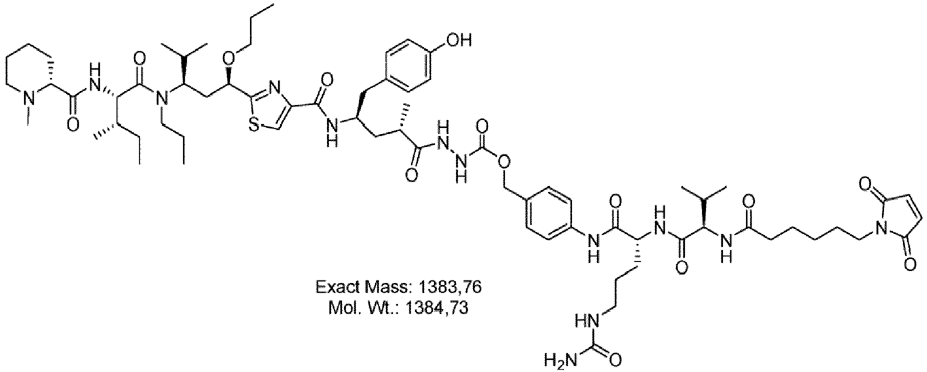

In accordance with this and other aspects of the present invention p may, in some cases, lie in the range 1 to 5, e.g. 1 to 4, or 1 to 3. In particular cases p may be 1 or 2. In particular, cases p may be 3 or 4.

In some embodiments, the conjugate is the conjugate described herein as OMTX705. OMTX705 comprises a humanised anti-FAP mAb (OMTX005) conjugated to the cytolysin TAM470 via a proteasecleavable vcPABA-(EG)₃ optimised linker. OMTX705 is described in Fabre et al. (2020).

In some embodiments, the sarcoma is a FAP-expressing (FAP+) leiomyosarcoma. In certain cases the conjugate is for use in the treatment of a leiomyosarcoma selected from the group consisting of: uterine leiomyosarcoma, cutaneous leiomyosarcoma, gastrointestinal leiomyosarcoma, stomach leiomyosarcoma, small intestine leiomyosarcoma, retroperitoneal leiomyosarcoma or abdominal leiomyosarcoma.

In some embodiments, the sarcoma is a FAP+ rhabdomyosarcoma. In some embodiments, the conjugate is for use in the treatment of a rhabdomyosarcoma selected from the group consisting of: embryonal rhabdomyosarcoma, alveolar rhabdomyosarcoma, spindle cell rhabdomyosarcoma, mixed-type rhabdomyosarcoma, pleomorphic rhabdomyosarcoma, and rhabdomyosarcoma with ganglionic differentiation.

In some embodiments, the sarcoma is a FAP+ Undifferentiated Pleomorphic Sarcoma (UPS).

In some embodiments, the conjugate is administered intravenously.

In some embodiments, the conjugate is administered weekly. In some embodiments, the conjugate is administered every 2 weeks. In some embodiments, the conjugate is administered monthly.

In some embodiments, the conjugate is administered within a dose range of 0.1-30 mg/kg. In some embodiments, the conjugate is administered within a dose range of 10-30 mg/kg. In some embodiments, the conjugate is administered at 10 mg/kg. In some embodiments, the conjugate is administered at 20 mg/kg. In some embodiments, the conjugate is administered at 30 mg/kg.

In some embodiments, the conjugate is administered in 4 doses.

In some cases the conjugate for use in a method of treatment of sarcoma is for simultaneous, sequential or separate administration with one or more other antitumor drugs. The one or more other antitumor drugs comprise a cytotoxic chemotherapeutic agent or an anti-angiogenic agent or an immunotherapeutic agent. In some cases the one or more other antitumor drugs comprise Gemcitabine, Abraxane, bevacizumab, itraconazole, carboxyamidotriazole, an anti-PD-1 molecule or an anti-PD-L1 molecule (for example, nivolumab or pembrolizumab).

In a second aspect, the invention provides an *in vitro* method of selecting a subject that has been determined to have sarcoma for treatment with a FAP-targeted ADC, comprising measuring the level of FAP expression in a biological sample taken from the subject; determining that the level of FAP expression is above a threshold level, and selecting the subject for treatment with the FAP-targeted ADC. In some embodiments, the sarcoma is leiomyosarcoma. In some embodiments, the sarcoma is rhabdomyosarcoma. In some embodiments, the sarcoma is UPS. In some embodiments, the FAP-targeted ADC is OMTX705. In some embodiments, the subject is a mammalian subject. In some embodiments, the subject is a human. In some embodiments, the biological sample is taken from the site of the tumour. In some embodiments, the biological sample is a nucleic acid-containing cell-free sample, for example a plasma sample.

In third aspect, the present invention provides a method of treating a FAP+ sarcoma in a mammalian subject, comprising administering a therapeutically effective amount of a conjugate as defined in accordance with the first aspect of the invention to the subject in need thereof. In some embodiments, the method is for treating a FAP+ leiomyosarcoma. In some cases, the method may be for treating uterine leiomyosarcoma, cutaneous leiomyosarcoma, gastrointestinal leiomyosarcoma, stomach leiomyosarcoma, small intestine leiomyosarcoma, retroperitoneal leiomyosarcoma or abdominal leiomyosarcoma. In some embodiments, the method is for treating a FAP+ rhabdomyosarcoma. In some embodiments, the method may be for treating rhabdomyosarcoma selected from the group consisting of: embryonal rhabdomyosarcoma, alveolar rhabdomyosarcoma, spindle cell rhabdomyosarcoma, mixed-type rhabdomyosarcoma, pleomorphic rhabdomyosarcoma, and rhabdomyosarcoma with ganglionic differentiation. In some embodiments, the method is for treating a FAP+ UPS.

In fourth aspect, the present invention provides the use of an anti-Fibroblast Activating Protein α (FAP) antibody-cytolysin conjugate, or a pharmaceutically acceptable salt or solvate thereof, in the preparation of a medicament for treatment of a FAP+ sarcoma, wherein the antibody-cytolysin conjugate has the formula A-(L-D)p, wherein A is an anti-FAP antibody that selectively binds FAP, L is a linker, D is a drug comprising a cytolysin and p is 1 to 10. In some embodiments, the sarcoma is FAP+ leiomyosarcoma. In some embodiments, the sarcoma is FAP+ rhabdomyosarcoma. In some embodiments, the sarcoma is FAP+ UPS.

Any preferred feature of the antibody-cytolysin conjugate or of the sarcoma as described in the first and second aspects apply equally to the third and fourth aspects.

The present invention includes the combination of the aspects and preferred features described except where such a combination is clearly impermissible or is stated to be expressly avoided. These and further aspects and embodiments of the invention are described in further detail below and with reference to the accompanying examples and figures.

### Sequences

In the following sequences, VH and VL domains are underlined and CDRH/CDRL regions are in bold. Mutations leading to ADCC and CDC deficiency are shown in bold italics. Signal sequences (where applicable) are shown boxed.

### hu36 IgG1-HC - with signal sequence

METDTLLLWVLLLWVPGSTG

QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPPVAGPSVFLFPPKPKDTLMISRTPEVTCVVVDVSHEDPEVK
FNWYVDGVEVHNAKTKPREEQYNSTYRVVSVLTVLHQDWLNGKEYKCKVSNKGLPSSIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 1)

### hu36-IgG1-LC - with signal sequence:

METDTLLLWVLLLWVPGSTG 
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKRTVAAPSVFIFPPSDEQLKSGTASVVCLL
 
NNFYPREAKVQWKVDNALQSGNSQESVTEQDSKDSTYSLSSTLTLSKADYEKHKVYACEVTHQGLSSP
VTKSFNRGEC (SEQ ID NO: 2)
*hu36-IgG1-HC* - *without signal sequence:* 
QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSSASTKGPSVFPLAPSSK
STSGGTAALGCLVKDYFPEPVTVSWNSGALTSGVHTFPAVLQSSGLYSLSSVVTVPSSSLGTQTYICNVN
HKPSNTKVDKKVEPKSCDKTHTCPPCPAPPVAGPSVFLFPPKPKDTLMISRTPEVTCVVVDVSHEDPEVK
FNWYVDGVEVHNAKTKPREEQYNSTYRVVSVLTVLHQDWLNGKEYKCKVSNKGLPSSIEKTISKAKGQP
REPQVYTLPPSRDELTKNQVSLTCLVKGFYPSDIAVEWESNGQPENNYKTTPPVLDSDGSFFLYSKLTVD
KSRWQQGNVFSCSVMHEALHNHYTQKSLSLSPGK (SEQ ID NO: 3)
*hu36-IgG1-LC* - *without signal sequence:* 
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKRTVAAPSVFIFPPSDEQLKSGTASVVCLL
NNFYPREAKVQWKVDNALQSGNSQESVTEQDSKDSTYSLSSTLTLSKADYEKHKVYACEVTHQGLSSP
VTKSFNRGEC (SEQ ID NO: 4)
*hu36- VH:* 
QVQLVQSGAEVKKPGASVKVSCKASGYTFTENIIHWVRQAPGQGLEWMGWFHPGSGSIKYNEKFKDRV
TMTADTSTSTVYMELSSLRSEDTAVYYCARHGGTGRGAMDYWGQGTLVTVSS (SEQ ID NO: 5)
*hu36- VL:* 
DIQMTQSPSSLSASVGDRVTITCRASKSVSTSAYSYMHWYQQKPGKAPKLLIYLASNLESGVPSRFSGS
GSGTDFTLTISSLQPEDFATYYCQHSRELPYTFGQGTKLEIKR (SEQ ID NO: 6)
*hu36-CDRH1:*
   ENIIH (SEQ ID NO: 7)
*hu36-CDRH2:*
   WFHPGSGSIKYNEKFKD (SEQ ID NO: 8)
*hu36-CDRH3:*
   HGGTGRGAMDY (SEQ ID NO: 9)
*hu36-CDRL1:*
   RASKSVSTSAYSYMH (SEQ ID NO: 10)
*hu36-CDRL2:*
   LASNLES (SEQ ID NO: 11)
*hu36-CDRL3:*
   QHSRELPYT (SEQ ID NO: 12)

### Human FAP

Also known as Seprase, 170 kDa melanoma membrane-bound gelatinase, fibroblast activation protein alpha or integral membrane serine protease. The amino acid sequence is disclosed at UniProt accession No. Q12884 (Version 140, dated 11 December 2013):

MKTWVKIVFGVATSAVLALLVMCIVLRPSRVHNSEENTMRALTLKDILNGTFSYKTFFPNWISGQEYLHQS
ADNNIVLYNIETGQSYTILSNRTMKSVNASNYGLSPDRQFVYLESDYSKLWRYSYTATYYIYDLSNGEFVR
GNELPRPIQYLCWSPVGSKLAYVYQNNIYLKQRPGDPPFQITFNGRENKIFNGIPDWVYEEEMLATKYAL
WWSPNGKFLAYAEFNDTDIPVIAYSYYGDEQYPRTINIPYPKAGAKNPVVRIFIIDTTYPAYVGPQEVPVPA
MIASSDYYFSWLTWVTDERVCLQWLKRVQNVSVLSICDFREDWQTWDCPKTQEHIEESRTGWAGGFFV
STPVFSYDAISYYKIFSDKDGYKHIHYIKDTVENAIQITSGKWEAINIFRVTQDSLFYSSNEFEEYPGRRNIY
RISIGSYPPSKKCVTCHLRKERCQYYTASFSDYAKYYALVCYGPGIPISTLHDGRTDQEIKILEENKELENA
LKNIQLPKEEIKKLEVDEITLWYKMILPPQFDRSKKYPLLIQVYGGPCSQSVRSVFAVNWISYLASKEGMVI
ALVDGRGTAFQGDKLLYAVYRKLGVYEVEDQITAVRKFIEMGFIDEKRIAIWGWSYGGYVSSLALASGTG
LFKCGIAVAPVSSWEYYASVYTERFMGLPTKDDNLEHYKNSTVMARAEYFRNVDYLLIHGTADDNVHFQ
NSAQIAKALVNAQVDFQAMWYSDQNHGLSGLSTNHLYTHMTHFLKQCFSLSD (SEQ ID NO: 13)

### Murine FAP

Also known as fibroblast activation protein alpha or integral membrane serine protease. Amino acid sequence is disclosed at UniProt accession No. P97321 (Version 117, dated 11 December 2013):

MKTWLKTVFGVTTLAALALVVICIVLRPSRVYKPEGNTKRALTLKDILNGTFSYKTYFPNWISEQEYLHQSE
DDNIVFYNIETRESYIILSNSTMKSVNATDYGLSPDRQFVYLESDYSKLWRYSYTATYYIYDLQNGEFVRG
YELPRPIQYLCWSPVGSKLAYVYQNNIYLKQRPGDPPFQITYTGRENRIFNGIPDWVYEEEMLATKYALW
WSPDGKFLAYVEFNDSDIPIIAYSYYGDGQYPRTINIPYPKAGAKNPVVRVFIVDTTYPHHVGPMEVPVPE
MIASSDYYFSWLTWVSSERVCLQWLKRVQNVSVLSICDFREDWHAWECPKNQEHVEESRTGWAGGFF
VSTPAFSQDATSYYKIFSDKDGYKHIHYIKDTVENAIQITSGKWEAIYIFRVTQDSLFYSSNEFEGYPGRRNI
YRISIGNSPPSKKCVTCHLRKERCQYYTASFSYKAKYYALVCYGPGLPISTLHDGRTDQEIQVLEENKELE
NSLRNIQLPKVEIKKLKDGGLTFWYKMILPPQFDRSKKYPLLIQVYGGPCSQSVKSVFAVNWITYLASKEGI
VIALVDGRGTAFQGDKFLHAVYRKLGVYEVEDQLTAVRKFIEMGFIDEERIAIWGWSYGGYVSSLALASG

TGLFKCGIAVAPVSSWEYYASIYSERFMGLPTKDDNLEHYKNSTVMARAEYFRNVDYLLIHGTADDNVHF
QNSAQIAKALVNAQVDFQAMWYSDQNHGISSGRSQNHLYTHMTHFLKQCFSLSD (SEQ ID NO: 14)

### Summary of the Figures

Embodiments and experiments illustrating the principles of the invention will now be discussed with reference to the accompanying figures in which:
**Figure 1****.** FAP staining of fibrosarcoma and leiomyosarcoma histological subtypes. **(A)** Fibrosarcoma with negative FAP staining. **(B)** Leiomyosarcoma with high level of FAP staining in tumour cells. FAP detected with DAB (brown) on hematoxylin-stained slides. Scale bar 500 µm.
**Figure 2****:** FAP staining of PDX leiomyosarcoma model SA4033. **(A)** Negative control and **(B)** positive immunohistochemical staining of FAP in sections of PDX tumour from leiomyosarcoma model SA4033. Scale bar 2 mm.
**Figure 3****.** Antitumoral effect of OMTX705 in a humanized PDX leiomyosarcoma cancer model after 4 weeks of treatment. **(A)** Mice bearing SA4033 PDX tumors were treated with Vehicle, OMTX705 monotherapy Group 1 with 10 mg/kg and Group 2 with 30 mg/kg, i.v., QWx 4 weeks (day 1, 8, 15, 22). **(B)** Evolution of body weight change (%). (C) Percentage of tumor inhibition volume in the different treatment groups.

### Detailed Description of the Invention

Aspects and embodiments of the present invention will now be discussed with reference to the accompanying figures. Further aspects and embodiments will be apparent to those skilled in the art. All documents mentioned in this text are incorporated herein by reference.

In describing the present invention, the following terms will be employed, and are intended to be defined as indicated below.

### FAP

As used herein "Fibroblast activation protein", "fibroblast activating protein", "FAP" and "FAPα" are used interchangeably. The FAP may be an FAP of any mammalian species. In some cases FAP is human FAP (also known as Seprase, 170 kDa melanoma membrane-bound gelatinase, fibroblast activation protein alpha or integral membrane serine protease), the amino acid sequence of which is disclosed at UniProt accession No. Q12884 (Version 140, dated 11 December 2013) (SEQ ID NO: 13). In some cases, a molecule that binds FAP (e.g. an antibody molecule or a conjugate thereof) may bind to a region of the extracellular domain of FAP. The extracellular domain of human FAP comprises residues 26-760 of the full-length human FAP protein. In some cases FAP is murine FAP (also known as fibroblast activation protein alpha or integral membrane serine protease), the amino acid sequence of which is disclosed at UniProt accession No. P97321 (Version 117, dated 11 December 2013) (SEQ ID NO: 14). The extracellular domain of murine FAP comprises residues 26-761 of the full-length murine FAP protein.

### Conjugate

As used herein "conjugate" includes the resultant structure formed by linking molecules and specifically includes antibody-drug conjugates (ADCs) and immunotoxins (ITs).

### Selectively binds

The terms selectively binds and selective binding refer to binding of an antibody, or binding fragment thereof, to a predetermined molecule (e.g. an antigen) in a specific manner. For example, the antibody, or binding fragment thereof, may bind to FAP, e.g. an extracellular portion thereof, with an affinity of at least about 1×10⁷M⁻¹, and may bind to the predetermined molecule with an affinity that is at least two-fold greater (e.g. five-fold or ten-fold greater) than its affinity for binding to a molecule other than the predetermined molecule.

### Antibody molecule

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb) and polyclonal antibodies (including polyclonal antisera). Antibodies may be intact or fragments derived from full antibodies (see below). Antibodies may be human antibodies, humanised antibodies or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule. The term "antiserum" or "antisera" refers to blood serum containing antibodies obtained from immunized animals.

It has been shown that fragments of a whole antibody can perform the function of binding antigens. Thus reference to antibody herein, and with reference to the methods, arrays and kits of the invention, covers a full antibody and also covers any polypeptide or protein comprising an antibody binding fragment. Examples of binding fragments are (i) the Fab fragment consisting of V_{L}, V_{H}, C_{L} and C_{H}1 domains; (ii) the Fd fragment consisting of the V_{H} and C_{H}1 domains; (iii) the Fv fragment consisting of the V_{L} and V_{H} domains of a single antibody; (iv) the dAb fragment which consists of a V_{H} domain; (v) isolated CDR regions; (vi) F(ab')₂ fragments, a bivalent fragment comprising two linked Fab fragments (vii) single chain Fv molecules (scFv), wherein a V_{H} domain and a V_{L} domain are linked by a peptide linker which allows the two domains to associate to form an antigen binding site; (viii) bispecific single chain Fv dimers (WO 93/11161) and (ix) "diabodies", multivalent or multispecific fragments constructed by gene fusion (WO94/13804; 58). Fv, scFv or diabody molecules may be stabilised by the incorporation of disulphide bridges linking the VH and VL domains. Minibodies comprising a scFv joined to a CH3 domain may also be made.

In relation to an antibody molecule, the term "selectively binds" may be used herein to refer to the situation in which one member of a specific binding pair will not show any significant binding to molecules other than its specific binding partner(s). The term is also applicable where e.g. an antigen-binding site is specific for a particular epitope that is carried by a number of antigens, in which case the specific binding member carrying the antigen-binding site will be able to bind to the various antigens carrying the epitope.

In some cases in accordance with the present invention the antibody may be a fully human antibody.

### Cytotoxic chemotherapeutic agents

In some cases in accordance with any aspect of the present invention, the conjugate of the invention may administered with, or for administration with, (whether simultaneously, sequentially or separately) one or more other antitumor drugs, including, but not limited to, a cytotoxic chemotherapeutic agent or an anti-angiogenic agent or an immunotherapeutic agent.

Cytotoxic chemotherapeutic agents are well known in the art and include anti-cancer agents such as:
Alkylating agents including nitrogen mustards such as mechlorethamine (HN2), cyclophosphamide, ifosfamide, melphalan (L-sarcolysin) and chlorambucil; 10 ethylenimines and methylmelamines such as hexamethylmelamine, thiotepa; alkyl sulphonates such as busulfan; nitrosoureas such as carmustine (BCNU), lomustine (CCNLJ), semustine (methyl-CCN-U) and streptozoein (streptozotocin); and triazenes such as decarbazine (DTIC; dimethyltriazenoimidazolecarboxamide);

Antimetabolites including folic acid analogues such as methotrexate (amethopterin); pyrimidine analogues such as fluorouracil (5- fluorouracil; 5-FU), floxuridine (fluorodeoxyuridine; FUdR) and cytarabine (cytosine arabinoside); and purine analogues and related inhibitors such as mercaptopurine (6-mercaptopurine; 6-MP), thioguanine (6-thioguanine; TG) and pentostatin (2'-deoxycofonnycin). Natural Products including vinca alkaloids such as vinblastine (VLB) and vincristine; epipodophyllotoxins such as etoposide and teniposide; antibiotics such as dactinomycin (actinomycin D), daunorabicin (daunomycin; rubidomycin), doxorubicin, bleomycin, plicamycin (mithramycin) and mitomycin (mitomycin Q; enzymes such as L-asparaginase; and biological response modifiers such as interferon alphenomes. Miscellaneous agents including platinum coordination complexes such as cisplatin (cis-DDP) and carboplatin; anthracenedione such as mitoxantrone and antbracycline; substituted urea such as hydroxyurea; methyl hydrazine derivative such as procarbazine (N- methylhydrazine, MIH); and adrenocortical suppressant such as mitotane (o, p'-DDD) and aminoglutethimide; taxol and analogues/derivatives; and hormone agonists/antagonists such as flutamide and tamoxifen. A further preferred cytotoxic agent is Gemcitabine (Gemzar^{®}). A further preferred cytotoxic agent is Paclitaxel bound to human serum albumin (Abraxane^{®}).

Anti-angiogenic agents are well known in the art and include anti-cancer agents such as bevacizumab, itraconazole, and carboxyamidotriazole.

Immunotherapeutic agents are known in the art and include, for example, anti-programmed cell death protein 1 (PD-1) antibodies and anti-programmed death-ligand 1 (PD-L1) antibodies, including Nivolumab (MDX1106) and Pembrolizumab (MK-3475).

### Pharmaceutical compositions

The conjugates of the present invention may be comprised in pharmaceutical compositions with a pharmaceutically acceptable excipient.

A pharmaceutically acceptable excipient may be a compound or a combination of compounds entering into a pharmaceutical composition which does not provoke secondary reactions and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

Conjugates of the present invention will usually be administered in the form of a pharmaceutical composition, which may comprise at least one component in addition to the conjugate. Thus pharmaceutical compositions may comprise, in addition to the conjugate, a pharmaceutically acceptable excipient, carrier, buffer, stabilizer or other materials well known to those skilled in the art. Such materials should be non-toxic and should not interfere with the efficacy of the conjugate. The precise nature of the carrier or other material will depend on the route of administration, which may be by bolus, infusion, injection or any other suitable route, as discussed below.

For intra-venous administration, for example by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN^{™}, PLURONICS^{™} or polyethylene glycol (PEG).

### Subject

The subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human. In some cases, the subject may be a human diagnosed with or classified as being at risk of developing a cancer, e.g., an epithelial tumor. In certain cases, the subject may be a laboratory animal, e.g., a mouse model of a cancer. In certain cases, the subject may be a mammal (e.g. a human) that has been diagnosed with or classified as being at risk of developing an inflammatory condition, such as rheumatoid arthritis (RA). In particular, the subject may be a human having RA.

### Cancer

The anti-FAP conjugates described herein find use in the treatment of a tumor in a mammalian subject. The tumor may be a solid tumor. The tumour may be a FAP+ sarcoma. The FAP+ sarcoma may be a leiomyosarcoma. In particular, the tumour may be a uterine leiomyosarcoma, cutaneous leiomyosarcoma, gastrointestinal leiomyosarcoma, stomach leiomyosarcoma, small intestine leiomyosarcoma, retroperitoneal leiomyosarcoma or abdominal leiomyosarcoma. The FAP+ sarcoma may be a rhabdomyosarcoma. In particular, the rhabdomyosarcoma may be embryonal rhabdomyosarcoma, alveolar rhabdomyosarcoma, spindle cell rhabdomyosarcoma, mixed-type rhabdomyosarcoma, pleomorphic rhabdomyosarcoma, and rhabdomyosarcoma with ganglionic differentiation. The FAP+ sarcoma may be Undifferentiated Pleomorphic Sarcoma (UPS).

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

The following is presented by way of example and is not to be construed as a limitation to the scope of the claims.

### Examples

### MATERIALS AND METHODS

### Clinical samples

Patients affected by different types of bone and soft tissue sarcomas were selected to construct the commercial soft tissue sarcoma microarray (TMA). A full description can be found on the website of US Biomax, Inc. (https://www.biomax.us/tissue-arrays/Soft_Tissue/SO809c and https://www.biomax.us/tissue-arrays/Bone_Cartilage/OS802c) including clinicopathological characteristics from the patients.

### Immunohistochemistry

Immunohistochemistry (IHC) detection of FAP was performed on a DISCOVERY ULTRA research instrument (F. Hoffman-La Roche, "Roche") using the rabbit monoclonal antibody against human FAP (1:25 dilution, Abcam-Ref. ab207178,) diluted in Anti-body Diluent Buffer (Antibody diluent, Ventana - Ref. 760-108). The staining protocol included a standard antigen retrieval step with Tris-EDTA buffer pH 8, incubation with the primary antibody for 1 h at 37 °C and incubation with the secondary antibody (Roche Omni Map Anti-rabbit, Ventana-Ref.760-4311) for 20 min at 37°C. Secondary antibody was detected by DAB (ChrommoMap DAB kit, Ventana-Ref.760-159).

### Scoring of immunohistochemistry and automated H-scoring

The intensity of FAP staining was analysed in tumour stroma compartment and tumour epithelium. FAP intensity was scored on an optical 3-point intensity scale (0 to 2) by an independent pathologist. Intensity average values were calculated from both compartments scores to obtain a case value. These three groups were subsequently reduced to two groups ("negative" and "positive").

FAP-stained TMA was scanned with scanner Olympus dotSlide version 2.1 at resolution 2 pixels per 1 µm. Digital image analysis procedure was described in detail previously (Mezheyeuski et al. (2018)). In brief, the FAP intensity was evaluated based on the marker expression level and quantified as number of positive cells per region unit. Analyses were performed in the stromal compartment and H-score was used to classify the samples, considering ranges from 0 to 300. H-score combines intensity and percentage of stained cells (Id et al. (2021)). H-score mean value was calculated to divide the sample in two groups ("Low H-score" and "High-score").

The binarized FAP positive/negative and H-score low/high classification of the cases were used in the patient with clinic-pathological characteristics.

### EXAMPLE 1 - Leiomyosarcoma patients express high levels of FAP

As shown in **Table 1A,** the population displays a cohort with a wide range of diagnosis from 17 to 78 years, 46.4 % females and 57.1 % males. FAP status showed significant association with the different bone and soft tissue tumour types *(p>0.001),* but not with age or gender (*p=0.662 and p=0.662).* All fibrosarcoma samples were unexpectedly classified FAP negative *(100* %) whereas a high percentage of FAP positivity (75%) was displayed by leiomyosarcoma group.

Based on the 3-point intensity scale, further analysis explored FAP positivity and pathology diagnosis **(Table 1B).** Interestingly, higher FAP intensity showed a significant correlation with leiomyosarcoma histology: indeed, 83.3 % of the FAP positive samples diagnosed as leiomyosarcoma were scored as high expression levels, and a significant association between high FAP intensity and leiomyosarcoma histologic subgroup was evidenced (p=0.015). Digital H-scoring classification similarly identified high FAP expression in most of the leiomyosarcoma samples studied **(Table 1C)** with significant results (p=0.007).

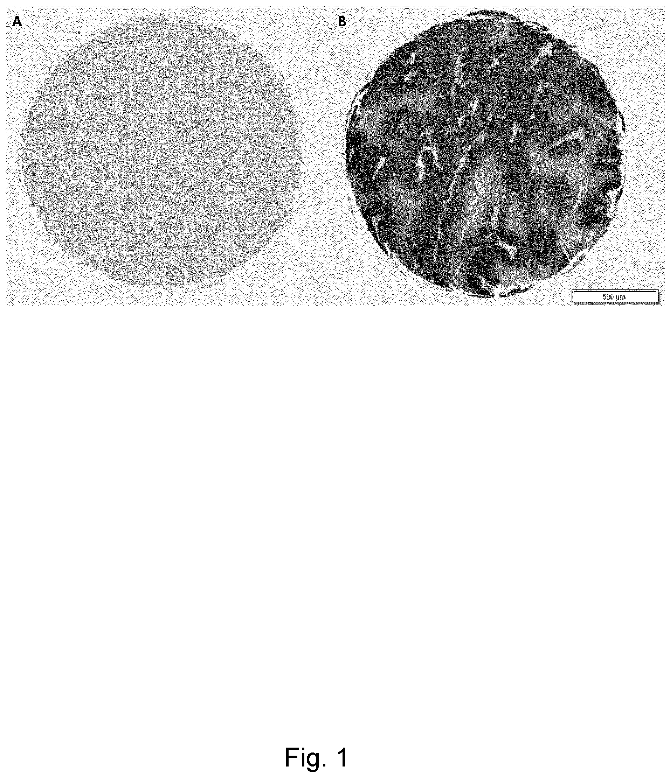

Representative cases of FAP-negative and FAP-positive staining are shown in **Figure 1****,** including fibrosarcoma FAP-negative core **(****Figure 1A****)** and high FAP intensity of leiomyosarcoma core **(****Figure 1B****).**

**Table 1A: FAP positivity and clinicopathological variables in fibrosarcoma and leiomyosarcoma patients (Chi-square exact test). Samples were categorised as negative or positive using visual scoring by a pathologist.**

| Variables | FAP staining (n=28) | |
|---|---|---|
| | Negative (n= 13) | Positive (n=15) |
| **Age (17 - 78)** | | *p*=*0.662* |
| < 47 | 5 | 7 |
| > or 47 | 8 | 8 |
| **Gender** | | *p*=*0.662* |
| Male | 8 | 8 |
| Female | 5 | 7 |
| **Pathology diagnosis** | | *p<0.001* |
| Fibrosarcoma | 8 | 0 |
| Leiomyosarcoma | 5 | 15 |

**Table 1B - Frequency of FAP intensity in fibrosarcoma and leiomyosarcoma histologic subtypes (Chi-square exact test).**

| Variables | FAP intensity (n=28) | | |
|---|---|---|---|
| | Negative or Low (n= 16) | Medium (n=2) | High (n=10) |
| **Pathology diagnosis** | | | *P=0.015* |
| Fibrosarcoma | 8 | 0 | 0 |
| Leiomyosarcoma | 8 | 2 | 10 |

**Table 1C - Frequency of FAP H-score in fibrosarcoma and leiomyosarcoma histologic subtypes (Chi-square exact test).**

| Variables | FAP H-score (n=28) | |
|---|---|---|
| | Low H-score (n= 17) | High H-score (n=11) |
| **Pathology diagnosis** | | *P*=*0.007* |
| Fibrosarcoma | 8 | 0 |
| Leiomyosarcoma | 9 | 11 |

Regarding the whole cohort of bone and soft tumour tissue samples, leiomyosarcoma showed the highest frequency of high FAP intensity (50%) compared to osteosarcoma (the largest histologic group in the study): only 7.69 % of osteosarcoma samples presented high FAP intensity (Table 2). Digital H-scoring also identified leiomyosarcoma as the largest subset within the high FAP expression group (66.67%) **(Table 3),** showing significant association between high FAP intensity and different bone and soft tissue tumours sub-histological groups (p<0.001). Among the top 15 groups of FAP H-score with highest values, leiomyosarcoma patients were the most represented (60%) **(Table 4).**

The data also highlights rhabdomyosarcoma as having substantial FAP expression, with 50% of the rhabdomyosarcoma samples having medium or high FAP intensity **(Table 2).** This trend is reflected in the digital H-scoring, which identified 60% of rhabdomyosarcoma samples as having medium or high FAP intensity **(Table 3).**

**Table 2: Correlation between intensity of FAP with bone and soft sarcoma subtypes (Chi-square exact test).**

| Variables | FAP intensity (n=132) | | |
|---|---|---|---|
| | Negative or Low (n= 90) | Medium (n=27) | High (n=15) |
| **Pathology diagnosis** | | | *p<0.001* |
| Osteosarcoma | 26 | 10 | 3 |
| Chondrosarcoma | 7 | 5 | 1 |
| Dermatofibrosarcoma | 10 | 0 | 0 |
| Fibrosarcoma | 8 | 0 | 0 |
| Leiomyosarcoma | 8 | 2 | 10 |
| Liposarcoma | 19 | 1 | 0 |
| Rhabdomyosarcoma | 10 | 9 | 1 |
| Malignant fibrohistiocytoma | 2 | 0 | 0 |

**Table 3: Correlation between digital H-score of FAP with bone and soft sarcoma subtypes (Chi-square exact test).**

| Variables | H-Score (n=149) | | |
|---|---|---|---|
| | Negative or Low (n= 106) | Medium (n=31) | High (n=12) |
| **Pathology diagnosis** | | | *p<0.001* |
| Osteosarcoma | 37 | 10 | 2 |
| Chondrosarcoma | 17 | 3 | 0 |
| Dermatofibrosarcoma | 7 | 3 | 0 |
| Fibrosarcoma | 7 | 1 | 0 |
| Leiomyosarcoma | 9 | 3 | 8 |
| Liposarcoma | 20 | 0 | 0 |
| Rhabdomyosarcoma | 8 | 10 | 2 |
| Malignant fibrohistiocytoma | 1 | 1 | 0 |

**Table 4: Pathology diagnosis of the top 15 samples with the highest H-Score.**

| **Pathology diagnosis** | **H-score** |
|---|---|
| Leiomyosarcoma | 297.61 |
| Leiomyosarcoma | 270.29 |
| Osteosarcoma | 269.42 |
| Leiomyosarcoma | 265.73 |
| Rhabdomyosarcoma | 264.01 |
| Rhabdomyosarcoma | 253.04 |
| Leiomyosarcoma | 237.33 |
| Leiomyosarcoma | 233.84 |
| Leiomyosarcoma | 228.20 |
| Osteosarcoma | 210.37 |
| Leiomyosarcoma | 206.45 |
| Leiomyosarcoma | 200.14 |
| Leiomyosarcoma | 194.76 |
| Rhabdomyosarcoma | 192.22 |
| Osteosarcoma | 191.67 |

### EXAMPLE 2 - Anti-tumoral efficacy of OMTX705 in a Leiomyosarcoma PDX mouse model

Due to leiomyosarcoma having the highest incidence of all sarcoma subtypes, comprising up to 20% of all sarcomas, and the lack of effective treatment (Kannan et al. (2022), Kasper et al. (2022)), we further investigated the anti-tumoral efficacy of OMTX705 in this subtype using a FAP positive patient-derived xenograft (PDX) model.

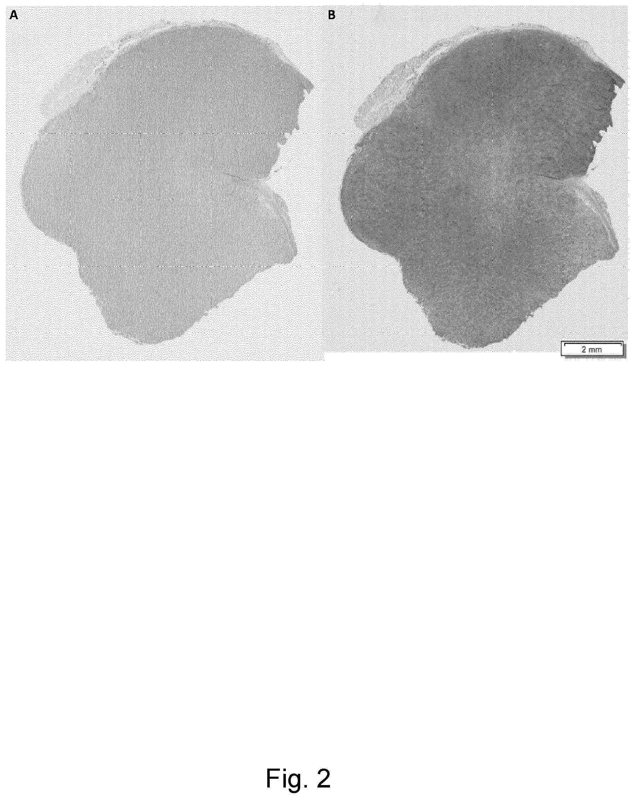

The PDX mouse model was selected from a preliminary study, using IHC FAP staining on FFPE sections from 12 different leiomyosarcoma tumour types. PDX model SA4033 was selected for its high FAP expression **(****Figure 2****).**

6-9-week-old female NOD/SCID mice were implanted subcutaneously with primary human tumour xenograft tumour fragment (approximately 2-3 mm in diameter) for tumour development (model SA4033). After tumour inoculation, the animals were checked daily for morbidity and mortality. When tumours reached a volume of approximately 150 mm³, mice were randomly allocated into groups (n=10/group) and treated intravenously with two different doses of OMTX705 (10 and 30 mg/kg) once a week for 4 weeks **(Table 5).**

**Table 5: Treatment plan for SA4033 Model Study. ROA: route of administration; i.v.: intravenous; BIW: Twice a week; QW: once a week.**

| **Group** | **Mice n^{o}** | **Treatment** | **Dose Level (mg/kg)** | **Dosing solution (mg/mL)** | **ROA** | **Dosing frequency & Duration** |
|---|---|---|---|---|---|---|
| 1 | 10 | Vehicle | - | - | *i.v.* | BIW (at day 1,4,8,11,15,18,22,25) |
| 2 | 10 | OMTX705 | 10 | 1 | *i.v.* | QW x 4 weeks |
| 2 | 10 | OMTX705 | 30 | 3 | *i.v.* | QW x 4 weeks |

During routine monitoring, the animals were checked for any effects of tumour growth and treatments on behaviour such as mobility, food and water consumption, body weight gain/loss (Body weights were measured twice per week after randomization), eye/hair matting and any other abnormalities. Mortality and observed clinical signs were recorded for individual animals in detail.

Tumour volumes were measured twice per week after randomization in two dimensions using a calliper, and the volume was expressed in mm³ using the formula: V = (L x W x W)/2, where V is tumour volume, L is tumour length (the longest tumour dimension) and W is tumour width (the longest tumour dimension perpendicular to L). Dosing as well as tumour and body weight measurements were conducted in a Laminar Flow Cabinet. After treatment end at Day 21, the post treatment observation period was extended 6 weeks for the group of mice treated with OMTX705 at 10 mg/kg.

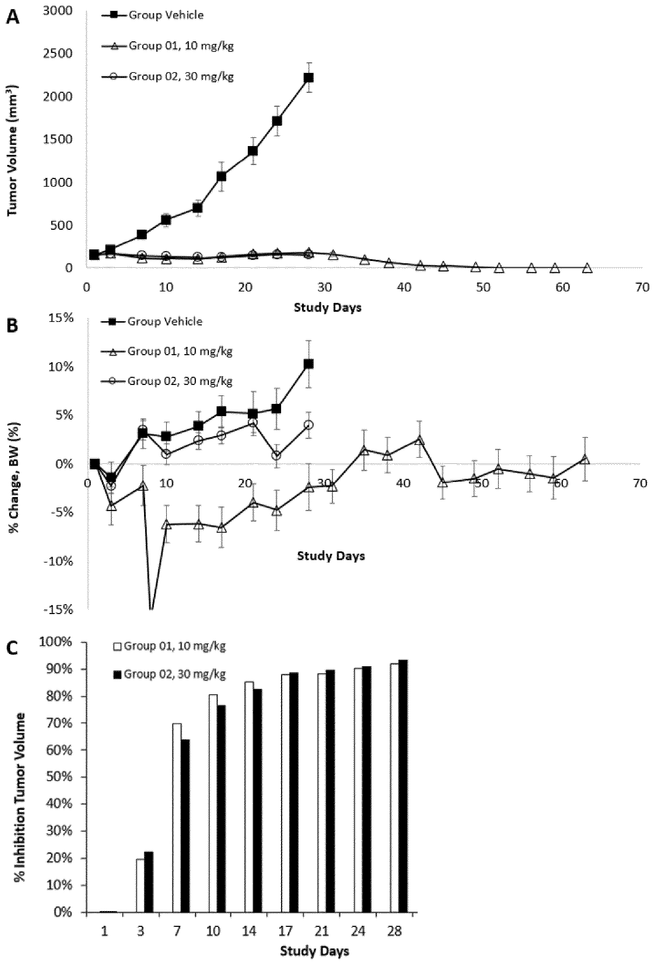

In the SA4033 PDX model of leiomyosarcoma, OMTX705 efficacy was measured *in vivo* as single agent administration at doses of 10 and 30 mg/kg i.v., once weekly for four doses. Tumor growth inhibition was found to be 92.04% and 93.11%, respectively **(****Figure 3A****).** Interestingly, in this model for leiomyosarcoma, OMTX705 as single agent at a dose of 30 mg/kg had a similar anti-tumoral effect compared to the lower dose (10 mg/kg), with no significant weight loss observed. Mice treated with doses of 10 and 30 mg/kg of OMTX705 presented less than 4% of body weight change per group during the study **(****Figure 3B****).** Percentage of tumor inhibition volume (TIV) of OMTX705 as single agent in this model is shown in **Figure 3C****.**

Upon post treatment observation at day 42, continuous tumor inhibition and even tumor regression was observed in the group treated with OMTX705 at 10 mg/kg, with mean tumor volume of 29.4 mm³, much lower than the average of tumor volumes registered at Day 0 (148.09 mm³). At day 42, 4 of the 10 animals were tumor-free. By the end of the post-treatment observation period, 100% of mice in this group were tumour-free. Therefore, these data demonstrate the long-lasting effect of a 10mg/kg weekly dose of OMTX705, even 6 weeks after treatment end.

### Summary

Previous results obtained in the First-in-Human Phase I study of ABBV-085, a new ADC targeting LRRC15 stroma protein, for treatment in sarcomas, presented a high response rate independently of sarcoma subtypes, due to overall LRRC15 expression in many solid tumours (Demetri et al. (2021), Purcell et al. (2022)). Further experiments to study LRRC15 expression and ABBV-085 activity using PDX models of soft-tissue sarcomas, demonstrated significant antitumor activity in undifferentiated sarcomas (UPS), dedifferentiated liposarcomas and leiomyosarcomas (Ben-Ami et al (2020)).

Indeed, unexpectedly, our study found highly FAP (+) leiomyosarcomas among other soft tissue sarcomas (i.e. liposarcomas or fibrosarcomas with very low or no FAP expression), thus identifying leiomyosarcoma subtype as a new and highly specific indication within sarcomas for OMTX705 antitumoral treatment.

The surprising finding that leiomyosarcoma tumour cells express FAP at a high level highlights the opportunity for treating leiomyosarcoma by targeting the cells directly with an anti-FAP-cytolysin conjugate. The *in vivo* data shown in **Figure 3** shows excellent tumour inhibition of 92.04% and 93.11% at doses of 10 and 30 mg/kg i.v respectively. Moreover, OMTX705 treatment actually resulted in tumour regression at later time points, with 100% of mice reaching tumour-free status. Thus, the data provides a new mechanism of action for OMTX705 besides targeting FAP (+) CAFs, as shown originally in the context of pancreatic cancer (Fabre et al. (2020)).

Both scoring systems (visual and digital), reached the same conclusion: the sarcoma subtype with semitotal and intense FAP staining was leiomyosarcoma, followed far behind by osteosarcoma.

The data also reveals rhabdomyosarcoma as having significant FAP expression, thus identifying a further target for an anti-FAP-cytolysin conjugate.

This experimental data is the first preclinical evidence that FAP targeting with an antibody-drug conjugate would be a promising strategy in FAP-positive sarcoma patients.

The findings presented in this study thus support a novel strategy and a new potential indication for OMTX705 therapeutic compound for the treatment of FAP+ sarcoma patients, such as leiomyosarcoma patients, with highly promising anti-tumoral efficacy compared to previous antibody-drug conjugate tested.

Furthermore, these results further support the use of FAP as biomarker for the selection of patients with FAP+ sarcoma, such as leiomyosarcoma soft tissue tumour subtype, with higher potential of response to the newly developed antibody-drug conjugate drug, OMTX705.

### References

A number of publications are cited above in order to more fully describe and disclose the invention and the state of the art to which the invention pertains. Full citations for these references are provided below. The entirety of each of these references is incorporated herein.
Acharyya, S., et al., Cell, 2012, 150: 165-178
Amer, K. et al., Journal of Orthopaedic Research (2019), 37:2226-2230; DOI 10.1002/jor.24387
Ben-Ami, E. et al. LRRC15 Targeting in Soft-Tissue Sarcomas: Biological and Clinical Implications.
Cancers 2020, Vol. 12, Page 75712, 757 (2020).
Brocks B., et al., Molecular Medicine, 2001, 7: 461-469
Crawford, Y., et al. Cancer Cell, 2009, 15: 21-34
Cormier et al. Soft tissue sarcomas. CA. Cancer J. Clin. 54, 94-109 (2004).
Demetri, G. D. et al. First-in-human phase I study of ABBV-085, an antibody-drug conjugate targeting LRRC15, in sarcomas and other advanced solid tumors. Clin. Cancer Res. 27, 3556-3566 (2021).
Erez, N., et al., Cancer Cell, 2010, 17: 135-147
Fabre, M. et al. OMTX705, a Novel FAP-Targeting ADC Demonstrates Activity in Chemotherapy and Pembrolizumab-Resistant Solid Tumor Models. (2020) doi:10.1158/1078-0432.CCR-19-2238.
Guedes, A., Oliveira, M. B. dos R., Costa, F. M. & Melo, A. S. de. Updating on Bone and Soft Tissue Sarcomas Staging. Rev. Bras. Ortop. 56, 411 (2021).
Gupta, G.P., et al., Cell, 2006: 127: 679-695
Hanahan, D., et al., Cancer Cell, 2012, 21: 309-322
Horimoto, Y., et al., Cell Adhes. Migr., 2012, 6: 193202
Hu, M., et al., Proc. Natl. Acad. Sci. USA, 2009, 106: 3372-3377
Hwang, R.F., et al., Cancer Res., 2008, 68: 918-926
Id, S. R. et al. Pixelwise H-score: A novel digital image analysis-based metric to quantify membrane biomarker expression from immunohistochemistry images. (2021) doi:10.1371 /journal.pone.0245638.
JN, C. & RE, P. Soft tissue sarcomas. CA. Cancer J. Clin. 54, 94-109 (2004).
Joyce, J.A., et al., Nat. Rev. Cancer, 2009, 9: 239-252
Kakarla, S., Song, X. T. & Gottschalk, S. Cancer-associated fibroblasts as targets for immunotherapy. Immunotherapy 4, 1129-1138 (2012).
Kalluri, R., Nat. Rev. Cancer, 2006, 6: 392-401
Kannan, S. et al. Leiomyosarcoma in the extremities and trunk wall: systematic review and meta-analysis of the oncological outcomes. World J. Surg. Oncol. 20, 124 (2022).
Kasper, B. et al. What Clinical Trials Are Needed for Treatment of Leiomyosarcoma? Curr. Treat. Options Oncol. 23, 439 (2022).
Kessler, L. et al. 68Ga-FAPI as a Diagnostic Tool in Sarcoma: Data from the 68Ga-FAPI PET Prospective Observational Trial. J. Nucl. Med. 63, 89-95 (2022).
Koerber, S. A. et al. Novel FAP ligands enable improved imaging contrast in sarcoma patients due to FAPI-PET/CT. Eur. J. Nucl. Med. Mol. Imaging (2021) doi:10.1007/S00259-021-05374-4.
Kratochwil, C. et al. 68Ga-FAPI PET/CT: Tracer uptake in 28 different kinds of cancer. J. Nucl. Med. 60, 801-805 (2019).
Kuyumcu, S., Sanli, Y. & Subramaniam, R. M. Fibroblast-Activated Protein Inhibitor PET/CT: Cancer Diagnosis and Management. Front. Oncol. 11, 1-10 (2021).
Kwan, T. T. et al. Abstract LBA032: Pan-cancer analysis of fibroblast activation protein alpha (FAP) expression to guide tumor selection for the peptide-targeted radionuclide therapy FAP-2286. Mol. Cancer Ther. 20, LBA032-LBA032 (2021).
Malanchi, I., et al., Nature, 2012, 481: 85-89
Meads, M.B, et al., Nat. Rev. Cancer, 2009, 9: 665-674
Medema, J.P., et al., Nature, 2011, 474: 318-326
Mersmann M., et al., Int. J. Cancer, 2001, 92: 240-248
Messerschmidt, S.K., et al., J Control Release, 2009, 137: 69-77
Mezheyeuski, A. et al. Multispectral imaging for quantitative and compartment-specific immune infiltrates reveals distinct immune profiles that classify lung cancer patients. J. Pathol. 244, 421-431 (2018).
Niederhuber, J., Armitage, J., Doroshow, J., Kastan, M. & Tepper, J. Abeloffs Clinical Oncology (5th ed.). Fac. Bookshelf (2014).
Nieman, K.M., et al., Nat. Med., 2011, 17: 1498-1503
Olive, K.P., et al., Science, 2009, 324: 1457-1461
Olumi, A.F., et al., Cancer Res., 1999, 59: 5002-5011
Orimo, A., et al., Cell, 2005, 121: 335-348
Ostermann E., et al., Clin. Cancer Res., 2008, 14: 4584-4592
Pietras, K., et al., Exp. Cell Res., 2010, 316: 1324-1331
Purcell, J. W. et al. Translational Science LRRC15 Is a Novel Mesenchymal Protein and Stromal Target for Antibody-Drug Conjugates. doi:10.1158/0008-5472.CAN-18-0327 (2022).
Ramirez-Montagut, T. et al. FAPα, a surface peptidase expressed during wound healing, is a tumor suppressor. Oncogene 2004 2332 23, 5435-5446 (2004).
Rettig, W. J. et al. Cell-surface glycoproteins of human sarcomas: differential expression in normal and malignant tissues and cultured cells. Proc. Natl. Acad. Sci. U. S. A. 85, 3110-3114 (1988).
Rettig, W. J. et al. Fibroblast activation protein: purification, epitope mapping and induction by growth factors. Int. J. cancer 58, 385-392 (1994).
Rettig, W. J. et al. Regulation and Heteromeric Structure of the Fibroblast Activation Protein in Normal and Transformed Cells of Mesenchymal and Neuroectodermal Origin. Cancer Res. 53, (1993).
Schmidt A., et al., Eur. J. Biochem., 2001, 268: 1730-1738
Shi, M., et al., World J. Gastroenterology, 2012, 28: 840-846.
Simková, A., Busek, P., Sedo, A. & Konvalinka, J. Molecular recognition of fibroblast activation protein for diagnostic and therapeutic applications. Biochimica et Biophysica Acta - Proteins and Proteomics vol. 1868 (2020).
Straussman, R., Nature, 2012, 487: 500-504
Strell, C., et al., Ups. J. Med. Sci., 2012, 117: 187-195
Valastyan, S., et al., Cell, 2011, 147: 275-292
Weinberg, R.A., et al., Garland science, Taylor & Francis Group LLC, New York, NY, USA, 2007
Xin, L. et al. Fibroblast Activation Protein-a as a Target in the Bench-to-Bedside Diagnosis and Treatment of Tumors: A Narrative Review. Front. Oncol. 11, (2021).
Yang, G., et al., Proc. Natl. Acad. Sci. USA, 2006, 103: 16472-16477
Zhang, L. et al. The role of fibroblast activation protein in progression and development of osteosarcoma cells. Clin. Exp. Med. 2019 201 20, 121-130 (2019).

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press.

## Claims

1. An anti-Fibroblast Activating Protein α (FAP) antibody-cytolysin conjugate having the formula A-(L-D)ₚ, or a pharmaceutically acceptable salt or solvate thereof, wherein A is an anti-FAP antibody that selectively binds FAP, L is a linker, D is a drug comprising a cytolysin and p is 1 to 10, for use in a method of treatment of sarcoma in a mammalian subject, wherein the sarcoma is **characterised by** FAP expression in the tumour.

2. The conjugate for the use according to claim 1, wherein the conjugate is the conjugate described herein as OMTX705.

3. The conjugate for the use according to claim 1 or 2, wherein the sarcoma is:
i) a FAP-expressing (FAP+) leiomyosarcoma, optionally wherein the leiomyosarcoma is selected from the group consisting of: uterine leiomyosarcoma, cutaneous leiomyosarcoma, gastrointestinal leiomyosarcoma, stomach leiomyosarcoma, small intestine leiomyosarcoma, retroperitoneum leiomyosarcoma, and/or abdominal wall leiomyosarcoma;
ii) a FAP+ rhabdomyosarcoma, optionally wherein the rhabdomyosarcoma is selected from the group consisting of: embryonal rhabdomyosarcoma, alveolar rhabdomyosarcoma, spindle cell rhabdomyosarcoma, mixed-type rhabdomyosarcoma, pleomorphic rhabdomyosarcoma, and rhabdomyosarcoma with ganglionic differentiation; or
iii) a FAP+ Undifferentiated Pleomorphic Sarcoma (UPS).

4. The conjugate for the use according to any preceding claim, wherein the conjugate is administered intravenously; optionally wherein the conjugate is administered weekly, every 2 weeks, or monthly; optionally wherein the conjugate is administered within a dose range of 0.1-30 mg/kg.

5. An *in vitro* method of selecting a subject that has been determined to have sarcoma for treatment with an anti-FAP ADC, comprising:
a) measuring the level of FAP expression in a biological sample taken from the subject;
b) determining that the level of FAP expression is above a threshold level, and
c) selecting the subject for treatment with the FAP-targeted ADC.

6. The *in vitro* method of claim 5, wherein the sarcoma is selected from the group consisting of leiomyosarcoma, rhabdomyosarcoma, and Undifferentiated Pleomorphic Sarcoma (UPS).

7. The *in vitro* method of claim 5 or 6, wherein the anti-FAP ADC is OMTX705.

8. The *in vitro* method of any of claims 5 to 7, wherein the subject is a mammalian subject, optionally wherein the mammalian subject is a human.

9. The conjugate for the use according to any of claims 1 to 4, wherein the anti-FAP antibody is a monoclonal antibody or binding fragment thereof that selectively binds to an extracellular region of human FAP and/or murine FAP;
optionally wherein the anti-FAP antibody comprises heavy chain complementarity determining regions 1-3 (CDRH1-3) and light chain complementarity determining regions 1-3 (CDRL1-3) having the following amino acid sequences:
CDRH1: SEQ ID NO: 7;
CDRH2: SEQ ID NO: 8;
CDRH3: SEQ ID NO: 9;
CDRL1: SEQ ID NO: 10;
CDRL2: SEQ ID NO: 11; and
CDRL3: SEQ ID NO: 12.

10. The conjugate for the use according to claim 9, wherein the anti-FAP antibody comprises a heavy chain variable region (VH), comprising the amino acid sequence of SEQ ID NO: 5 and a light chain variable region (VL) comprising the amino acid sequence of SEQ ID NO: 6;
or wherein the anti-FAP antibody comprises a heavy chain comprising the amino acid sequence of SEQ ID NO: 3, and a light chain comprising the amino acid sequence of SEQ ID NO: 4.

11. The conjugate for the use according to any of claims 1 to 4 or 9 to 10, wherein the cytolysin comprises formula IV: 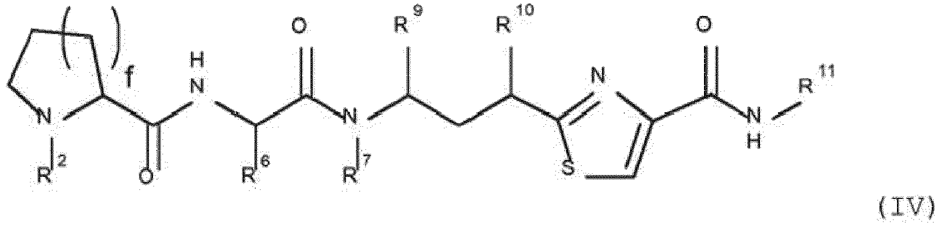 wherein:
R² is H or C₁-C₄ alkyl;
R⁶ is C₁-C₆ alkyl;
R⁷ is C₁-C₆ alkyl, CH₂OR¹⁹ or CH₂OCOR²⁰, wherein R¹⁹ is alkyl, R²⁰ is C₂-C₆-alkenyl, phenyl, or CH₂-phenyl;
R⁹ is C₁-C₆ alkyl;
R¹⁰ is H, OH, O-alkyl or O-acetyl;
f is 1 or 2;
R¹¹ has the following structure: 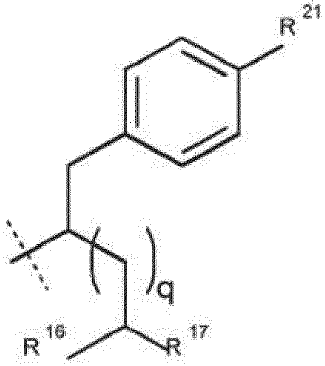
wherein
R²¹ is H, OH, halogen, NH₂, alkyloxy, phenyl, alkyl amino or dialkyl amino;
R¹⁶ is H or a C₁-C₆-alkyl group;
R¹⁷ is directly or indirectly attached to linker L; and
q is 0, 1, 2 or 3;
and wherein the term "optionally substituted" relates to groups, wherein one or several H atoms can be replaced by F, Cl, Br or I or OH, SH, NH₂, or NO₂; the term "optionally substituted" further relates to groups, which can be exclusively or additionally substituted with unsubstituted C₁-C₆ alkyl, C₂C₆ alkenyl, C₂-C₆ alkynyl, C₁-C₆ heteroalkyl, C₃-C₁₀ cycloalkyl, C₂-C₉ heterocycloalkyl, C₆-C₁₀ aryl, C₁-C₉ heteroaryl, C₇-C₁₂ aralkyl or C₂-C₁₁ heteroaralkyl groups.

12. The conjugate for the use according to any of claims 1 to 4 or 9 to 11, wherein L comprises a spacer, optionally wherein the spacer comprises -(OCH₂CH₂)ₙ-, wherein n is 2 to 5.

13. The conjugate for the use according to any of claims 1 to 4 or 9 to 12, wherein L comprises an attachment group for attachment to A; optionally wherein L comprises a protease cleavable portion comprising a valine-citrulline unit.

14. The conjugate for the use according to any one of claims 11 to 13, wherein the cytolysin has the formula: 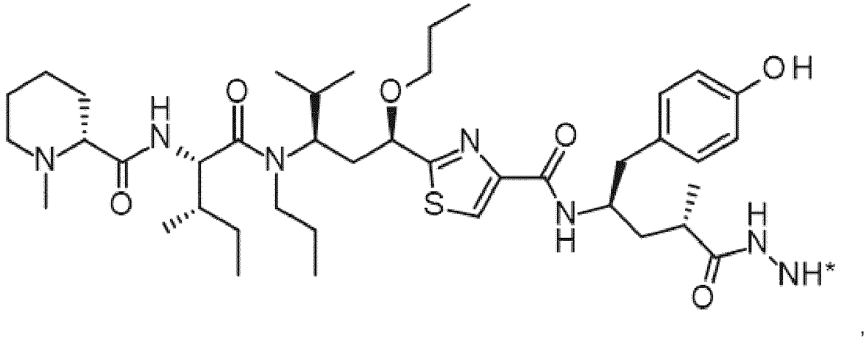 wherein * indicates the site of attachment to L.

15. The conjugate for the use according to any of claims 1 to 4 or 9 to 14, wherein -L-D has the structure:
